(19)

**Europäisches Patentamt**
**European Patent Office**
**Office européen des brevets**

(11) **EP 2 069 561 B1**

(12) **EUROPÄISCHE PATENTSCHRIFT**

(45) Veröffentlichungstag und Bekanntmachung des Hinweises auf die Patenterteilung:
**25.01.2012 Patentblatt 2012/04**

(21) Anmeldenummer: **07820776.8**

(22) Anmeldetag: **01.10.2007**

(51) Int Cl.:
*D01G 31/00* (2006.01)     *G01N 22/00* (2006.01)
*G01N 33/36* (2006.01)

(86) Internationale Anmeldenummer:
**PCT/EP2007/060392**

(87) Internationale Veröffentlichungsnummer:
**WO 2008/040703 (10.04.2008 Gazette 2008/15)**

(54) **VORRICHTUNG FÜR EINE TEXTILMASCHINE ZUR MESSUNG DER LÄNGENSPEZIFISCHEN MASSE UND/ODER DER FEUCHTIGKEIT EINES STRANGFÖRMIGEN, LAUFENDEN FASERGEMENGES SOWIE TEXTILMASCHINE**

DEVICE FOR A TEXTILE MACHINE FOR MEASURING THE LENGTH-SPECIFIC MASS AND/OR THE MOISTURE OF A STRAND-SHAPED, CONTINUOUS FIBER BATCH, AND TEXTILE MACHINE

DISPOSITIF POUR UNE MACHINE TEXTILE DESTINÉ À MESURER LA MASSE LINÉAIRE ET/OU L'HUMIDITÉ D'UNE QUANTITÉ DE FIBRE DÉFILANTE EN ÉCHEVEAU, AINSI QUE MACHINE TEXTILE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **02.10.2006 DE 102006046713**

(43) Veröffentlichungstag der Anmeldung:
**17.06.2009 Patentblatt 2009/25**

(73) Patentinhaber: **Rieter Ingolstadt GmbH**
**85055 Ingolstadt (DE)**

(72) Erfinder: **RUSSER, Peter**
**81929 München (DE)**

(74) Vertreter: **Schlief, Thomas P.**
**Patentanwälte**
**Canzler & Bergmeier**
**Friedrich-Ebert-Straße 84**
**85055 Ingolstadt (DE)**

(56) Entgegenhaltungen:
**DE-A1- 10 306 217     DE-A1- 10 313 964**
**DE-A1- 10 334 144     US-B1- 6 476 619**

**Beschreibung**

Die vorliegende Erfindung betrifft eine Vorrichtung für eine Textilmaschine zur Messung der längenspezifischen Masse und/oder der Feuchtigkeit eines strangförmigen, laufenden Fasergemenges. Eine erfindungsgemäße Vorrichtung ist insbesondere für eine Spinnereivorbereitungsmaschine, beispielsweise für eine Strecke, Karde oder Kämmmaschine oder für eine Spinnereimaschine, beispielsweise für eine Spinnmaschine oder Spulmaschine vorgesehen. Sie umfasst einen Mikrowellenresonator, welcher einen Messraum aufweist, durch den das Fasermenge hindurchführbar ist, und eine Anordnung zur Erfassung der Resonanzfrequenz und/oder der Güte des Mikrowellenresonators.

Weiterhin betrifft die vorliegende Erfindung eine Textilmaschine, insbesondere eine Spinnereivorbereitungsmaschine, beispielsweise eine Strecke, Karde oder Kämmmaschine oder eine Spinnereimaschine, beispielsweise eine Spinnmaschine oder Spulmaschine.

Im Betrieb einer Textilmaschine ist es häufig erforderlich, ein strangförmiges, laufendes Fasergemenge hinsichtlich seiner längenspezifischen Masse zu vermessen. Der Begriff "strangförmiges Fasergemenge" umfasst dabei beispielsweise Faserbänder, Garne oder Zwirne. Die längenspezifische Masse gibt dabei an, welche Masse das Fasergemenge pro Längeneinheit aufweist. Die Messergebnisse dieser Größe werden insbesondere zur Steuerung der jeweiligen Textilmaschine herangezogen. Weiterhin dienen derartige Messergebnisse zur Beurteilung der Qualität eines produzierten Fasergemenges.

Die bisher überwiegend eingesetzten, mechanisch abtastenden Sensoren zur Ermittlung der längenspezifischen Masse von laufenden Fasergemengen werden den Anforderungen an künftige Generationen von Textilmaschinen, insbesondere wegen der angestrebten Erhöhung der Arbeitsgeschwindigkeit, nicht mehr gerecht.

Eine neue Methode zur Messung der längenspezifischen Masse eines Fasergemenges stellt hingegen die Verwendung von Mikrowellen dar. Dabei wird das zu vermessende Fasergemenge durch einen Messraum eines Mikrowellenresonators kontinuierlich hindurchgeführt. Da die dielektrischen Eigenschaften textiler Fasergemenge von den dielektrischen Eigenschaften der Umgebungsluft abweichen, verändert sich durch das Hindurchführen des Fasergemenges sowohl die Resonanzfrequenz als auch die Güte des Mikrowellenresonators.

Zur Bestimmung der Resonanzfrequenz und der Güte eines Mikrowellenresonators ist es bekannt, ein mittels eines Mikrowellengenerators erzeugtes Mikrowellensignal mit einer variierenden Frequenz in den Mikrowellenresonator einzukoppeln. Üblicherweise wird dabei ein vorab festgelegter Frequenzbereich kontinuierlich oder in festgelegten Schritten durchfahren, wobei für jede Frequenz des eingekoppelten Mikrowellensignals die Amplitude der resultierenden Schwingung im Mikrowellenresonator erfasst wird.

Aus der so aufgenommenen Resonanzkurve kann dann sowohl die Resonanzfrequenz (das ist diejenige Frequenz, bei der die höchste Amplitude auftritt) als auch die Güte des Mikrowellenresonators ermittelt werden. Hierzu wird zunächst die Bandbreite des Mikrowellenresonators aus der Resonanzkurve bestimmt. Dabei gilt folgende Festsetzung: die Bandbreite ist jener Frequenzbereich der Resonanzkurve, in dem die Amplitude größer oder gleich dem $1/\sqrt{2}$ -ten Teil der maximalen Amplitude ist. Die Güte kann dann in einfacher Weise bestimmt werden, indem die Resonanzfrequenz durch die Bandbreite geteilt wird.

Bei der Produktion und/oder der Verarbeitung von textilen Fasergemengen ist vor allem die Trocken- bzw. die Substanzmasse des Fasergemenges von Bedeutung. Allerdings neigen textile Fasergemenge, insbesondere wenn sie Naturfasern, wie beispielsweise Baumwollfasern, enthalten, zur Aufnahme von Feuchtigkeit. Sowohl die Trockenmasse als auch die Feuchtigkeit beeinflussen die Resonanzfrequenz und die Güte des Mikrowellenresonators. Da dies jedoch in unterschiedlicher Weise geschieht, kann aus der gemessenen Resonanzfrequenz und aus der gemessenen Güte des Mikrowellenresonators sowohl die Trockenmasse des Fasergemenges als auch dessen Feuchtegehalt ermittelt werden. Zur Klarstellung sei angemerkt, dass der hier verwendete Begriff "längenspezifische Masse" sich auf die absolute längenspezifische Trockenmasse bezieht, während sich der Begriff "Feuchtigkeit" auf die absolute längenspezifische Masse des vom Fasergemenge aufgenommenen Wassers bezieht.

Bei dem bekannten Verfahren entspricht die Messzeit mindestens jener Zeit, welche für das Durchfahren des vorab festgelegten Frequenzbereichs benötigt wird. Dabei kann der Frequenzbereich allerdings nicht beliebig schnell durchfahren werden, da die Amplitude des Mikrowellenresonators bei einer Änderung der Frequenz des Oszillators erst nach Abklingen des sogenannten Einschwingvorgangs ihren endgültigen Wert annimmt. Die minimal erforderliche Messzeit kann beispielsweise bei einem linearen Frequenzhub abgeschätzt werden, indem der zu durchfahrende Frequenzbereich durch das Quadrat der Bandbreite des Mikrowellenresonators geteilt wird. Die erforderliche Messzeit erhöht sich jedoch noch weiter, wenn die Genauigkeitsanforderungen steigen.

Wenn jedoch die Messzeit so erhöht wird, dass Einschwingvorgänge keine wesentliche Rolle mehr spielen, so beziehen sich die Amplitudenwerte der ermittelten Resonanzkurve bei einem laufenden Fasergemenge auf unterschiedliche Abschnitte des selben. Dies wiederum führt zu Ungenauigkeiten, welche mit steigender Laufgeschwindigkeit des Fasergemenges zunehmen.

Die US 6,476,619 offenbart ein erstes Ausführungsbeispiel einer Vorrichtung zur Messung der längenspezifischen Masse und/oder der Feuchtigkeit eines strangförmigen, laufenden Fasergemenges. Im ersten Ausführungsbeispiel werden dazu wie oben beschrieben Mikrowellen einer steuerbaren Frequenz mittels eines Breitband-Generator-Synthesizers erzeugt und in den Mikrowellenresonator eingekoppelt. Dabei wird in einem Sweeping-Mode die Frequenz des Synthesizers periodisch variiert, so dass eine Resonanzkurve aufgenommen werden kann.

Weiterhin offenbart die US 6,476,619 ein zweites Ausführungsbeispiel einer Vorrichtung zur Messung der längenspezifischen Masse und/oder der Feuchtigkeit eines strangförmigen, laufenden Fasergemenges. Im zweiten Ausführungsbeispiel wird dazu die Resonanzfrequenz des Mikrowellenresonators erfasst und ausgewertet. Eine Erfassung der Güte des Mikrowellenresonators ist ausdrücklich nicht vorgesehen. Die dadurch bedingten Messunsicherheiten sollen durch einen zusätzlichen Feuchtesensor ausgeschaltet werden.

Nachteilig bei dem bekannten Verfahren ist es somit, dass, insbesondere bei einem schnell laufenden Fasergemenge, die gewünschte Genauigkeit der Erfassung der längenspezifischen Masse eines Fasergemenges und/oder dessen aufgenommener Feuchtigkeit prinzipiell nicht erreicht werden kann.

Aufgabe der vorliegenden Erfindung ist es somit, eine Vorrichtung für eine Textilmaschine bzw. eine Textilmaschine vorzuschlagen, bei welcher die genannten Nachteile vermieden sind. Insbesondere soll die Messzeit verringert und so die Genauigkeit der Erfassung der längenspezifischen Masse eines Fasergemenges und/oder dessen aufgenommener Feuchtigkeit verbessert werden.

Die Aufgabe wird gelöst durch die Merkmale der unabhängigen Ansprüche.

Eine erfindungsgemäße Vorrichtung weist einen Mikrowellenoszillator auf, der eine aktive Verstärkeranordnung und ein der Verstärkeranordnung zugeordnetes Rückkopplungsnetzwerk mit einer frequenzbestimmenden Resonanzanordnung aufweist. Das grundlegende Funktionsprinzip des so ausgebildeten Mikrowellenoszillator besteht darin, dass ein am Eingang des Verstärkers anliegendes Eingangssignal durch den Verstärker verstärkt und ein so erzeugtes, am Ausgang des Verstärkers anliegendes Signal über das Rückkopplungsnetzwerk an den Eingang des Verstärkers zurückgekoppelt wird. Ein derartiger Mikrowellenoszillator wird auch Rückkopplungsoszillator, Zweitoroszillator oder - in der älteren Literatur - Vierpoloszillator genannt. Eine Schwingung tritt jedoch nur dann auf, wenn sowohl die sogenannte Amplitudenbedingung als auch die sogenannte Phasenbedingung für die Entstehung einer Schwingung erfüllt sind.

Während die Amplitudenbedingung besagt, dass ein Oszillator nur dann schwingen kann, wenn der Verstärker die Abschwächung im Rückkopplungsnetzwerk aufhebt, also die Gesamtverstärkung von Verstärker und Rückkopplungsnetzwerk den Wert 1 annimmt, besagt die Phasenbedingung, dass eine Schwingung nur dann zustande kommen kann, wenn die Summe der Phasenverschiebungen im Verstärker und im Rückkopplungsnetzwerk einen der Werte ..., $-2\pi$, $0$, $+2\pi$, ... annimmt.

Um eine Schwingung mit einer definierten Frequenz zu erhalten, weist das Rückkopplungsnetzwerk eine Resonanzanordnung auf, welche bewirkt, dass die Amplitudenbedingung und die Phasenbedingung für eine bestimmte Frequenz, nämlich für eine sogenannte Resonanzfrequenz, erfüllt sind. Die Resonanzanordnung bestimmt damit auch die Frequenz des Mikrowellenoszillators.

Die frequenzbestimmende Resonanzanordnung ist dabei zu einer elektrischen Schwingung nach Art eines LC-Schwingkreises fähig. Sie kann daher in einem Ersatzschaltbild durch einen LC-Schwingkreis repräsentiert werden, der eine elektrische Induktivität, eine elektrische Kapazität sowie - zur Berücksichtigung von Verlusten - einen elektrischen Widerstand umfasst. Die Funktionsweise des Mikrowellenoszillators insgesamt entspricht damit der Funktionsweise eines LC-Oszillators.

Der prinzipielle Aufbau und die prinzipielle Funktionsweise eines LC-Oszillators sind beispielsweise in dem Buch U. Tietze, Ch. Schenk, Halbleiter-Schaltungstechnik, vierte Auflage, Berlin 1978 ab Seite 419 beschrieben.

Bei der erfindungsgemäßen Vorrichtung ist der Mikrowellenresonator die frequenzbestimmende Resonanzanordnung des Rückkopplungsnetzwerkes, so dass die Resonanzfrequenz des Mikrowellenresonators die Frequenz des Mikrowellenoszillators bestimmt.

Da die Resonanzfrequenz des Mikrowellenresonators von der längenspezifischen Masse des darin befindlichen Fasergemenges abhängig ist, können aus der Frequenz des Mikrowellenoszillators Rückschlüsse auf eben die längenspezifische Masse des im Messraum befindlichen Fasergemenges gezogen werden, ohne dass wie aus dem Stand der Technik bekannt, Resonanzkurven erfasst und ausgewertet werden müssten. Hierdurch verkürzt sich der Zeitbedarf zur Erzeugung eines Messwertes beträchtlich, so dass insbesondere mit steigender Laufgeschwindigkeit des Fasergemenges die Genauigkeit des jeweiligen Messwertes erhöht werden kann. Der einzelne Messwert wird nicht mehr durch die Bewegung des Abschnittes des Fasergemenges während der Messdauer verfälscht.

Einschränkend sei lediglich angemerkt, dass allein aus der Frequenz des Mikrowellenoszillators nur dann mit hinreichender Genauigkeit auf die längenspezifische Masse des Fasergemenges geschlossen werden kann, wenn dessen Feuchtigkeit bekannt ist. Dieser kann beispielsweise mit einem separaten Feuchtigkeitsmesser erfasst werden.

Vorteilhafterweise ist eine Frequenzmessanordnung zur Erzeugung eines mit der Frequenz des Mikrowellenoszillators korrespondierenden Frequenzsignals vorgesehen. Das erzeugte Frequenzsignal kann dann zur Steuerung einer Textilmaschine und/oder zur Erfassung der Qualität eines Fasergemenges herangezogen werden.

Je nach Ausführung der Einrichtung, für welche das Frequenzsignal zur weiteren Verarbeitung oder Nutzung vorgesehen ist, kann es vorteilhaft sein, wenn das Frequenzsignal als Analogsignal und/oder als Digitalsignal bereitgestellt ist.

In einem besonders bevorzugten Ausführungsbeispiel weist die aktive Verstärkeranordnung ein Amplitudenstellglied mit einer durch ein Amplitudenstellsignal einstellbaren Verstärkung auf. Dabei ist ein Amplitudendetektor zur Erzeugung des Amplitudenstellsignals vorgesehen. Das Amplitudenstellsignal wird dabei so erzeugt, dass die Gesamtverstärkung, welche die Verstärkung der aktiven Verstärkeranordnung (einschließlich des Amplitudenstellgliedes) und des Rückkopplungsnetzwerkes umfasst, konstant auf dem Wert 1 gehalten ist.

Das Amplitudenstellsignal korrespondiert dabei mit der Güte des Mikrowellenresonators. Dies liegt darin begründet, dass die Amplitude der Schwingung des Schwingkreises bei gegebener Anregung von dessen Güte abhängig ist. Mit steigender Güte steigt auch die Amplitude der Schwingung des Parallelschwingkreises und damit dessen Verstärkung. Dies führt dazu, dass die Verstärkung der aktiven Verstärkeranordnung abgesenkt werden muss, wenn die Gesamtverstärkung konstant auf dem Wert 1 gehalten werden soll. Verkehrt, wenn sich die Güte des Schwingkreises verringert, so sinkt auch die Amplitude der Schwingung im Schwingkreis und damit dessen Verstärkung, so dass die Verstärkung der Verstärkeranordnung erhöht werden muss. Es ist nun also möglich, die Güte des Mikrowellenresonators ohne aufwendige Aufnahme und Auswertung einer Resonanzkurve zu erfassen. Die durch das Amplitudenstellsignal repräsentierte Güte kann dann in bekannter Weise ausgewertet werden.

In einer vorteilhaften Ausführungsform ist der Amplitudendetektor zum Vergleichen der Amplitude eines mit dem Eingangssignal der aktiven Verstärkeranordnung korrespondierenden Amplitudensignals mit einem Sollwert ausgebildet, sodass das Eingangssignal mit konstanter Amplitude schwingt. In diesem Fall kann eine eingangsseitige Übersteuerung der aktiven Verstärkeranordnung ohne weiteres verhindert werden.

In einer weiteren vorteilhaften Ausführungsform ist der Amplitudendetektor zum Vergleichen der Amplitude eines mit dem Ausgangssignal der aktiven Verstärkeranordnung korrespondierenden Amplitudensignals mit einem Sollwert ausgebildet, sodass das Ausgangssignal mit konstanter Amplitude schwingt. In diesem Fall kann eine ausgangsseitige Übersteuerung der aktiven Verstärkeranordnung ohne weiteres verhindert werden.

Wenn die aktive Verstärkeranordnung in ihrem amplitudenlinearen Bereich betrieben ist, so ergibt sich ein linearer Zusammenhang zwischen dem Amplitudenstellsignal und der Güte des Mikrowellenresonators. Dies vereinfacht die Bestimmung der Güte.

Vorteilhafterweise ist vorgesehen, dass die aktive Verstärkeranordnung einen Verstärker mit konstanter Verstärkung aufweist, der mit dem Amplitudenstellglied in Reihe geschaltet ist. Hierdurch können die Funktion "Verstärkung" und die Funktion "Verstärkungsregelung" von jeweils hierzu optimierten Modulen durchgeführt werden.

Das Amplitudenstellsignal kann als Analogsignal und/oder als Digitalsignal erzeugt sein. Beispielsweise kann es als Analogsignal an ein analoges Amplitudenstellglied übertragen werden und gleichzeitig als Digitalsignal zur weiteren Auswertung bereitgestellt werden.

Bevorzugt ist eine Signalauswertestufe vorgesehen, welche aus dem Frequenzsignal und dem Amplitudenstellsignal Messwerte der längenspezifischen Masse und/oder Messwerte der Feuchtigkeit des strangförmigen, laufenden Fasergemenges erzeugt. Die so erzeugten Messwerte können unmittelbar zur Steuerung einer Textilmaschine und/oder zur Erfassung der Qualität des Fasergemenges verwendet werden.

Vorteilhafterweise ist der Mikrowellenoszillator so ausgebildet, dass die aktive Verstärkeranordnung im amplitudenlinearen Bereich betrieben ist. In diesem Fall vereinfacht sich die Regelung der Amplitude des Ausgangssignals der Verstärkeranordnung. Auch verbessert sich die Genauigkeit der jeweiligen Messungen. Insbesondere sind Messfehler durch Übersteuerung der aktiven Verstärkeranordnung vermieden.

In einem Ausführungsbeispiel weist der Mikrowellenresonator eine Koppelanordnung auf, welche den Messraum mit einem Eingangssignal und mit einem Ausgangssignal der aktiven Verstärkeranordnung verkoppelt. Der Vorteil liegt darin, dass lediglich eine Koppelanordnung benötigt wird. Ein Mikrowellenresonator, der lediglich eine Koppelanordnung aufweist, wird auch Eintorresonator genannt.

Vorteilhafterweise ist zur Verkopplung der Koppelanordnung mit dem Eingang und dem Ausgang der aktiven Verstärkeranordnung ein Übertrager vorgesehen. Neben einer derartigen induktiven Kopplung wäre aber auch eine Kopplung mittels Kapazitäten und/oder Widerständen möglich.

Mit Vorteil ist der Mikrowellenoszillator als Dreipunktoszillator ausgebildet. Dabei wird bevorzugt eine induktive Dreipunktschaltung verwendet. Die Dreipunktschaltung ist verhältnismäßig einfach aufgebaut und insbesondere dann geeignet, wenn ein Eintorresonator verwendet wird. Das Grundprinzip der induktiven Dreipunktschaltung ist beispielsweise in dem bereits erwähnten Buch von U. Tietze und Ch. Schenk ab Seite 423 erläutert.

In einem alternativen Ausführungsbeispiel ist der Mikrowellenresonator als Zweitorresonator ausgebildet. Er weist eine Einkoppelanordnung, über die das Ausgangssignal der Verstärkeranordnung in den Messraum eingekoppelt ist und eine Auskoppelanordnung, über die das Eingangssignal der Verstärkeranordnung aus dem Messraum ausgekoppelt ist, auf. Ein Mikrowellenresonator mit zwei Koppelanordnungen wird auch Zweitorresonator genannt.

Insbesondere wenn der Mikrowellenresonator als Zweitorresonator ausgebildet ist, kann der Mikrowellenoszillator als Meissner-Oszillator ausgebildet sein. Das Grundprinzip des Meissner-Oszillators ist beispielsweise in dem bereits mehrfach erwähnten Buch von U. Tietze und Ch. Schenk ab Seite 421 beschrieben.

Mit Vorteil weist der Amplitudendetektor eine Gleichrichterschaltung zum Gleichrichten des Amplitudensignals und einen Differenzverstärker zur Erzeugung des Amplitudenstellsignals anhand des gleichgerichteten Amplitudensignals-und anhand eines vorgebbaren Referenzsignals auf. Es ergibt sich ein vergleichsweise einfacher Aufbau des Amplitudendetektors.

Bei einem weiteren vorteilhaften Ausführungsbeispiel ist vorgesehen, dass der Amplitudendetektor eine Anordnung, vorzugsweise einen Richtkoppler, zum Auskoppeln einer phasengleichen Komponente (auch als Kophasalsignal bezeichnet) und einer um 90° phasenverschobenen Komponente (auch als Quadratursignal bezeichnet) aus dem Amplitudensignal eine erste Quadrierschaltung zum Quadrieren der phasengleichen Komponente, eine zweite Quadrierschaltung zum Quadrieren der um 90° phasenverschobenen Komponente, einen Additionsverstärker zum Erzeugen eines Proportionalsignals, welches proportional der Summe aus der quadrierten phasengleichen Komponente und der quadrierten phasenverschobenen Komponente ist und einen Differenzverstärker zur Erzeugung des Amplitudenstellsignals durch Vergleich des Proportionalsignals mit einem vorgebbaren Referenzsignal aufweist. Mittels einem derart ausgebildeten Amplitudendetektor kann eine schnellere Regelung der Gesamtverstärkung erreicht werden.

Vorteilhafterweise ist wenigstens ein FPGA vorgesehen ist. Bevorzugt ist vorgesehen, die Signalauswertestufe mittels eines entsprechend eingerichteten FPGAs zu realisieren. Es ist jedoch auch möglich, die Frequenzmessanordnung und/oder den Amplitudendetektor durch jeweils einen entsprechend modifizierten FPGA auszubilden. Vorteilhafterweise ist ein einzelner FPGA so eingerichtet, dass er die Funktion mehrerer oder aller der genannten Baugruppen übernimmt.

Bei einem FPGA (Abkürzung für "Field Programmable Gate Array") handelt es sich um einen modifizierbaren Logikbaustein, dessen Funktion durch spezifische Konfiguration interner Strukturen festlegbar ist. Daher können mit ein und demselben FPGA nahezu beliebige Schaltungen realisiert werden. Hierdurch ergibt sich ein Kostenvorteil im Vergleich zur Verwendung eine Anwendungsspezifischen Integrierten Schaltung (ASIC). Im Gegensatz zu einem Mikroprozessor, dessen Funktion durch ein sequentiell abzuarbeitendes Programm festgelegt werden muss, bietet der FPGA die Möglichkeit der parallelen Informationsverarbeitung, was zu einer höheren Verarbeitungsgeschwindigkeit führt.

Bevorzugt sind der Mikrowellenresonator und zumindest Teile der daran angeschlossenen Schaltung, insbesondere der Übertrager, der Verstärker, das Amplitudenstellglied, der eingangsseitige Abschlusswiderstand, der ausgangsseitige Abschlusswiderstand, die Frequenzmessanordnung, der Amplitudendetektor und/oder die Signalauswertestufe, in einem gemeinsamen Gehäuse angeordnet sind. Hierdurch kann der in einem textilen Umfeld erforderliche Schutz der Gesamtvorrichtung gegen Schmutz, Faserflug, Temperaturschwankungen, mechanische Einwirkungen usw. in einfacher Weise bewerkstelligt werden.

Mit Vorteil weist die Vorrichtung eine Kommunikationsschnittstelle mit einem definierten Protokoll zum Anbinden der Vorrichtung an eine Kommunikationsverbindung einer Textilmaschine auf. Moderne Textilmaschinen weisen eine Vielzahl steuerungsrelevanter Baugruppen auf. Dabei sind Kommunikationsverbindungen zum Verbinden dieser Baugruppen vorgesehen. Wenn also die erfindungsgemäße Vorrichtung eine nach einem entsprechenden Protokoll arbeitende Kommunikationsschnittstelle aufweist, so ermöglicht dies eine einfache Montage der Vorrichtung bei der Produktion einer Textilmaschine oder bei einer nachträglichen Umrüstung einer solchen. Bei Verwendung einer Bus-Schnittstelle kann der Verkabelungsaufwand minimiert werden. Besonders geeignet sind beispielsweise CAN-Bus-Schnittstellen.

Eine erfindungsgemäße Textilmaschine ist dadurch gekennzeichnet, dass wenigstens eine erfindungsgemäße Vorrichtung vorgesehen ist.

Weitere Vorteile der Erfindung sind in den nachfolgenden Ausführungsbeispielen beschrieben. Es zeigen:

**Figur 1** eine Strecke als Beispiel für eine Textilmaschine nach dem Stand der Technik;

**Figur 2** eine erfindungsgemäße Strecke;

**Figur 3** eine erfindungsgemäße Vorrichtung mit einem Eintor-Mikrowellenresonator;

**Figur 4** ein Ersatzschaltbild der Vorrichtung nach Figur 3;

**Figur 5** eine weitere erfindungsgemäße Vorrichtung mit einem Eintor-Mikrowellenresonator;

**Figur 6** ein Ersatzschaltbild der Vorrichtung nach Figur 5;

**Figur 7** eine erfindungsgemäße Vorrichtung mit einem Zweitor-Mikrowellenresonator;

**Figur 8** ein Ersatzschaltbild der Vorrichtung nach Figur 7;

**Figur 9** eine weitere erfindungsgemäße Vorrichtung mit einem Zweitor-Mikrowellenresonator;

**Figur 10** ein Ersatzschaltbild der Vorrichtung nach Figur 9;

**Figur 11** ein Ausführungsbeispiel eines Amplitudendetektors;

**Figur 12** ein weiteres Ausführungsbeispiel eines Amplitudendetektors; und

**Figur 13** eine beispielhafte Ausführung eines Mikrowellenresonators.

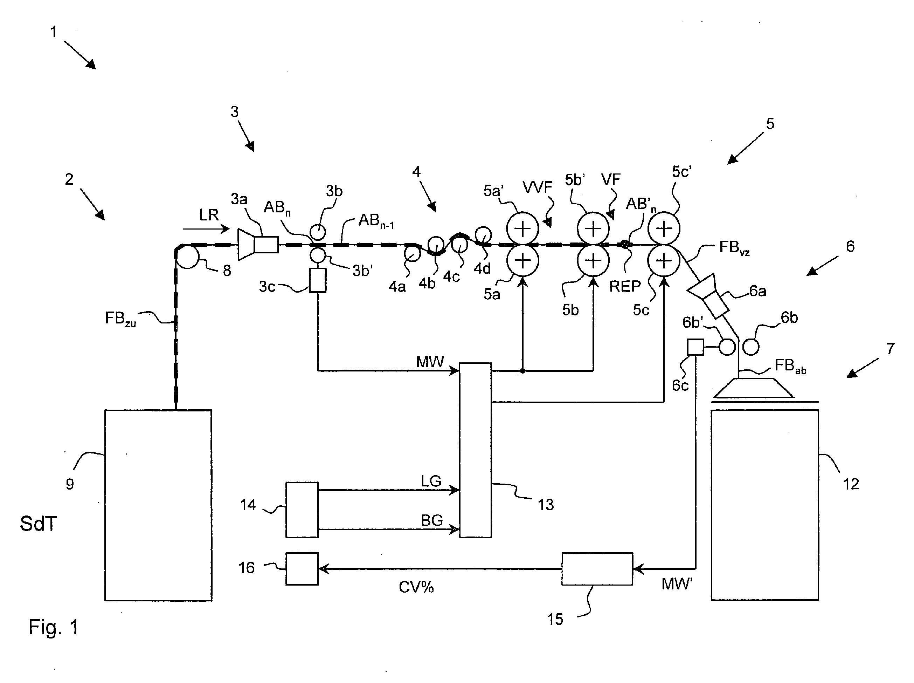

Figur 1 zeigt eine nach dem Stand der Technik ausgebildete Strecke 1, als Beispiel für eine Textilmaschine 1. Das der Strecke 1 vorgelegte Fasergemenge FB wird in Laufrichtung LR über eine Bandzuführung 2, eine Einlaufsensoreinheit 3, eine Umlenkeinheit 4, ein Streckwerk 5, eine Auslaufführung 6 und über eine Bandablage 7 geführt.

Die nur skizzenhaft dargestellte Bandzuführung 2 weist ein Umlenkrohr 8 auf, welches so angeordnet ist, dass das vorgelegte Fasergemenge $FB_{zu}$ in Form eines Faserbandes $FB_{zu}$ aus einer der Strecke 1 beigestellten Spinnkanne 9 entnommen werden kann. Die Bandzuführung 2 könnte jedoch auch so ausgebildet sein, dass sie ein vorgelegtes Faserband $FB_{zu}$ direkt von einer laufenden Karde übernehmen kann. Ebenfalls, und das ist in der Praxis der häufigste Fall, könnte die Bandzuführung 2 zur gleichzeitigen Entnahme mehrerer vorgelegter Faserbänder $FB_{zu}$ aus verschiedenen beigestellten Spinnkannen 9 ausgebildet sein. Wenn im folgenden von Faserband $FB_{zu}$ gesprochen wird, soll dadurch nicht ausgeschlossen werden, dass auch mehrere Faserbändern $FB_{zu}$ gemeint sind.

Das vorgelegte Faserband $FB_{zu}$ wird von der Bandzuführung 2 zu der Einzugseinheit 3 transportiert. Diese umfasst einen Einlauftrichter 3a und ein angetriebenes Tastwalzenpaar 3b, 3b', welches aus einer ortsfest gelagerten Walze 3b sowie einer beweglich gelagerten und belasteten Walze 3b' besteht.

Das Tastwalzenpaar 3b, 3b' dient einerseits dem Hindurchziehen des Faserbandes $FB_{zu}$ durch den Einlauftrichter 3a, um es so zu komprimieren und andererseits der abschnittsweisen Erfassung der längenspezifischen Masse des hindurchgeführten Faserbandes $FB_{zu}$. Zu letzterem ist ein Wegaufnehmer 3c zur Erfassung der Bewegung der beweglichen Walze 3b' vorgesehen. Letztlich wird somit der Querschnitt des Faserbandes $FB_{zu}$ erfasst und daraus auf die längenspezifische Masse des Faserbandes $FB_{zu}$ geschlossen. Im Einlaufbereich, also stromaufwärts des Streckwerks 5, erfolgt die Erfassung der längenspezifischen Masse des hindurchgeführten Fasergemenges $FB_{zu}$. Also durch mechanische Abtastung. Die einzelnen vermessenen Abschnitte AB weisen dabei eine Länge von einigen Millimetern auf. Für jeden vermessenen Abschnitt AB wird durch den Wegaufnehmer 3c ein Messwert MW erzeugt.

Die Umlenkeinheit 4 mit den Bandumlenkstäben 4a, 4b, 4c und 4d dient dazu, das von der Einzugseinheit 3 kommende Faserband $FB_{zu}$ quer zur Laufrichtung gleichmäßig auszubreiten und dabei die im Bereich der Einzugseinheit 3 erfolgte Pressung des Faserbandes $FB_{zu}$ aufzulösen.

Beim Transport des Faserbandes $FB_{zu}$ von der Einzugseinheit 3 zum Streckwerk 5 wird das Faserband $FB_{zu}$ einer Einzugsspannung unterworfen, welche durch eine unterschiedliche Umfangsgeschwindigkeit des Tastwalzenpaares 3a, 3a' und des Einzugswalzenpaares 5a, 5a' bewirkt wird.

Das Streckwerk 5 umfasst ein Eingangswalzenpaar 5a, 5a' sowie ein Mittelwalzenpaar 5b, 5b' und ein Lieferwalzenpaar 5c, 5c'. Die Walzenpaare 5a, 5a'; 5b, 5b'; 5c, 5c'; sind derart angetrieben, dass die Drehzahl von Walzenpaar zu Walzenpaar in Laufrichtung zu nimmt. Hierdurch wird das Faserband $FB_{zu}$ sowohl im Vorverzugsfeld VVF, welches zwischen dem Eingangswalzenpaar 5a, 5a' und dem Mittelwalzenpaar 5b, 5b' gebildet ist, als auch im Hauptverzugsfeld VF, welches zwischen dem Mittelwalzenpaar 5b, 5b' und dem Lieferwalzenpaar 5c, 5c' gebildet ist, verzogen.

Die Unterwalzen 5a, 5d, 5c des Streckwerkes 5 sind ortsfest angeordnet, hingegen sind die Oberwalzen 5a', 5b' 5c' beweglich gelagert und werden mittels nicht gezeigter Belastungsmittel gegen die Unterwalzen 5a, 5b, 5c gedrückt, so dass sich eine sichere Klemmung des Faserbandes $FB_{zu}$ ergibt.

Die Auslaufführung 6 umfasst einen Auslaufrichter 6a sowie ein angetriebenes Abzugswalzenpaar 6b, 6b', von denen eine Walze 6b ortsfest gelagert und die andere Walze 6b' beweglich gelagert ist. Der Auslauftrichter 6a bewirkt eine Komprimierung des verzogenen Faserbandes $FB_{vz}$ so dass ein kompaktes Faserband $FB_{ab}$ entsteht.

Die Abzugswalzen 6b, 6b' dienen zunächst dem Abziehen des Faserbandes $FB_{ab}$ aus dem Auslauftrichter 6a sowie der weiteren Kompaktierung des Faserbandes $FB_{ab}$. Darüber hinaus ist der beweglich gelagerten Walze 6b' ein weiterer Wegaufnehmer 6c zur Erzeugung von Messwerten MW', welche mit der längenspezifischen Masse des abgeführten Faserbandes $FB_{ab}$ korrespondiert, zugeordnet. Damit kommt bei herkömmlichen Strecken 1 auch im Auslaufbereich, also stromabwärts des Streckwerks 5, das Prinzip der mechanischen Abtastung zur Bestimmung der längenspezifischen Masse des abgeführten Fasergemenges $FB_{ab}$ zur Anwendung.

Die Bandablage 7, welche hier nicht im Detail erläutert wird, bewirkt die geordnete Ablage des mittels der Strecke 1 erzeugten Faserbandes $FB_{ab}$ in eine Spinnkanne 12.

Das Streckwerk 5 ist über eine Steuerungsvorrichtung 13 steuerbar. Dabei ist der Steuerungsvorrichtung 13 eine Bedieneinheit 14 zugeordnet, welche es einem Bediener ermöglicht, Einstellwerte vorzugeben, welche dann als Eingangsgröße an die Steuerungsvorrichtung 13 übermittelt werden. Derartige durch den Bediener vorgebbare Eingangsgrößen sind beispielsweise die gewünschte Liefergeschwindigkeit LG und das gewünschte Bandgewicht BG erzeugten Faserbandes $FB_{ab}$. Die Liefergeschwindigkeit LG ist dabei jene Geschwindigkeit, mit der die verzogenen Faserbänder FB das Streckwerk 5 verlassen. Das Bandgewicht BG hingegen beschreibt die durchschnittliche längenspezifische Masse des von der Strecke 1 abgegebenen Faserbandes $FB_{ab}$. Weitere Eingangsgröße der Steuerungsvorrichtung 13 ist der aktuelle Messwert MW, der von der Einlaufsensoreinheit 3 automatisch zur Steuerungsvorrichtung 13 übertragen wird. Dargestellt sind lediglich ausgewählte Eingangsgrößen der Steuerungsvorrichtung 13. In der Praxis werden der Steuerungsvorrichtung 13 weitere Eingangsgrößen zugeführt.

Die Steuerungsvorrichtung 13 ist so ausgebildet, dass sie in Abhängigkeit von ihren Eingangsgrößen die Drehzahl des Eingangswalzenpaares 5a, 5a', die Drehzahl des Mittelwalzenpaares 5b, 5b' und die Drehzahl des Lieferwalzenaares 5c, 5c' durch Einwirkung auf nicht gezeigte Antriebsmittel steuert. Im unregulierten Betrieb wird dabei für das Vorverzugsfeld VVF ein fester Vorverzug und für das Hauptverzugsfeld VF ein fester Hauptverzug festgelegt.

Im regulierten Betrieb hingegen sind Verzugsänderungen, auch Regeleingriffe genannt, vorgesehen, um das dem Streckwerk 5 zugeführte Faserband $FB_{zu}$ zu vergleichmäßigen. Derartige Regeleingriffe erfolgen auf der Basis der Messungen des Wegaufnehmers 3c. Eine Steuerung, bei welcher der Messort vor dem Streckwerk 5 liegt nennt man üblicherweise Open-Loop-Steuerung. Bei einer derartigen Steuerung ist die Laufstrecke bzw. die Laufzeit eines Abschnittes AB der zugeführten Faserband $FB_{zu}$ bis zu dem Punkt REP, an dem der Regeleingriff erfolgen soll, zu berücksichtigen. Laufstrecke und Laufzeit sind über die Einzugsgeschwindigkeit des Streckwerks miteinander verknüpft.

Das dem Verzugsfeld VF zugeführte Fasergemenge $FB_{zu}$ besteht aus nacheinander angeordneten Abschnitten. Durch das Bezugszeichen $AB_n$ ist der Abschnitt, der im dargestellten Moment durch die Sensoreinrichtung 3c vermessen wird, bezeichnet. Stromabwärts des Abschnitts $AB_n$ liegt der Abschnitt $AB_{n-1}$. Aus Vereinfachungsgründen sind die weiteren Abschnitte nicht durch Bezugszeichen benannt. Für jeden der Abschnitte wird wenigstens ein Messwert MW ermittelt, der mit der längenspezifischen Masse des jeweiligen Abschnitts AB korrespondiert und der an die Steuerungseinrichtung 13 übermittelt wird.

Wenn der vermessene Abschnitt $AB_n$ den Regeleinsatzpunkt REP, also die mit $AB'_n$ bezeichnete Position erreicht, wird durch die Steuerungseinrichtung 13 ein entsprechender Regeleingriff veranlasst. Weist der Abschnitt $AB_n$ beispielsweise eine über dem Durchschnitt liegende längenspezifische Masse auf, so wird eine Erhöhung des Verzugs zur Vergleichmäßigung des Fasergemenges FB eingeleitet.

Wesentlich für die Qualität der erzeugten Faserbandes $FB_{ab}$ ist insbesondere die Genauigkeit der Messwerte MW, welche die längenspezifische Masse des zugeführten Faserbandes $FB_{zu}$ abbilden. Dabei ist die Qualität um so höher, je gleichmäßiger das erzeugte Faserband $FB_{ab}$ ist.

Zur Überprüfung der Qualität der erzeugten Faserbandes $FB_{ab}$ ist es aus dem Stand der Technik bekannt, mittels einer Auswerteeinheit 15 aus den Messwerten MW' des Messtrichters 6a einen Variationskoeffizient zu errechnen, der die prozentuale Bandungleichmäßigkeit für eine bestimmte Bezugslänge des Faserbandes $FB_{ab}$ angibt. Die Bezugslänge wird auch Schnittlänge, der Variationskoeffizient auch CV%-Wert genannt. In der Praxis liegen die verwendeten Schnittlängen im Bereich zwischen einigen Zentimetern und einigen Metern. Verlässliche CV%-Werte können allerdings nur erhalten werden, wenn die Messwerte MW' eine hohe Genauigkeit aufweisen. Die ermittelten CV%-Werte werden beispielsweise an einer Anzeige 16 dargestellt oder zur späteren Auswertung abgespeichert.

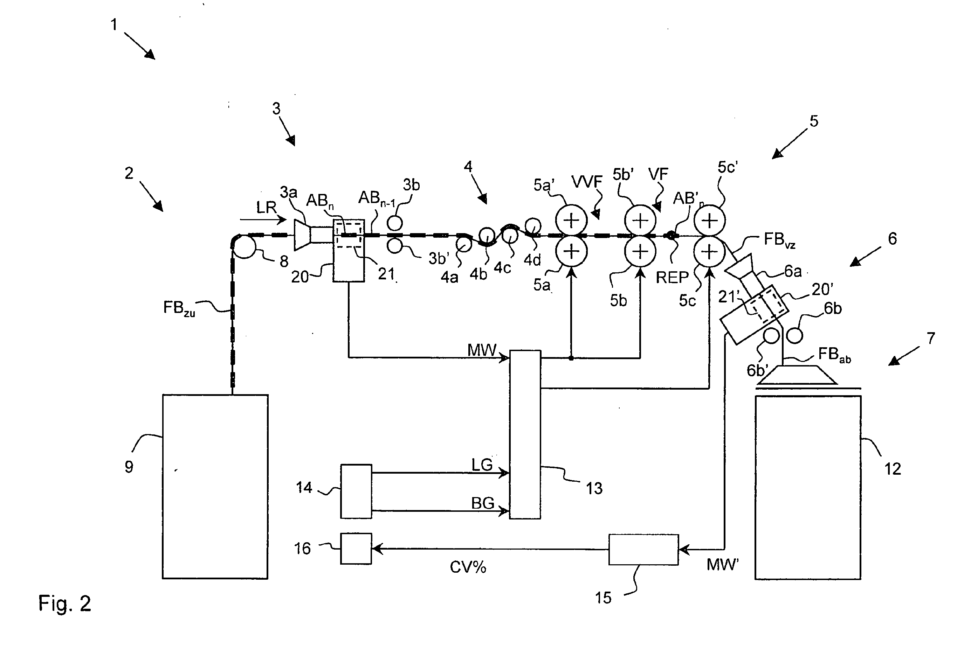

Figur 2 zeigt eine erfindungsgemäße Strecke 1 als Beispiel für eine erfindungsgemäße Textilmaschine 1. Sie zeichnet sich dadurch aus, dass eine erste erfindungsgemäße Vorrichtung 20 im Bereich der Einzugseinheit 3 und eine zweite erfindungsgemäße Vorrichtung 20' im Bereich der Auslaufführung 6 vorgesehen ist. Die erste erfindungsgemäße Vorrichtung 20 ersetzt den Wegaufnehmer 3c der in der Figur 1 dargestellten Strecke 1. Die Vorrichtung 20 dient dabei ebenfalls der Erfassung der längenspezifischen Masse der Abschnitte AB des dem Streckwerk 5 zugeführten Faserbandes $FB_{zu}$ sowie der Erzeugung von Messwerten, welche diese Informationen umfassen.

Die Messwerte MW werden in der bekannten Weise zur Steuerung der Strecke 1 verwendet. Die Erfassung der längenspezifischen Masse erfolgt dabei berührungslos unter Verwendung von Mikrowellen. Wegen der fehlenden Massenträgheit kann somit die Arbeitsgeschwindigkeit der Strecke prinzipiell erhöht werden. Die Erfassung der längenspezifischen Masse des Faserbandes FB erfolgt auf der Basis einer Auswertung der dielektrischen Eigenschaften des Messraumes 22 der erfindungsgemäßen Vorrichtung 20. Die mittels der Vorrichtung 20 erzeugten Messwerte MW bilden die längenspezifische Trockenmasse des Fasergemenges $FB_{zu}$ ab, so dass Fehler bei der Steuerung der Textilmaschine 1 durch Veränderungen des Feuchtegehaltes des Faserbandes $FB_{zu}$ vermieden sind. Da die Messzeit der erfindungsgemäßen Vorrichtung 20 gegenüber bekannten Mikrowellenmessvorrichtungen verringert ist, ergibt sich eine hohe Genauigkeit der Messwerte MW auch dann, wenn das Fasergemenge $FB_{zu}$ mit hoher Geschwindigkeit bewegt wird. Insgesamt wird eine Mittelung der längenspezifischen Masse mehrerer aufeinanderfolgender Abschnitte AB vermieden. Jeder Messwert MW repräsentiert genau einen Abschnitt AB.

Die weitere erfindungsgemäße Vorrichtung 20' erfasst abschnittsweise die längenspezifische Masse des abzulegenden Fasergemenges $FB_{ab}$. Die mit der längenspezifischen Masse korrespondierenden Messwerte MW' werden in bekannter Weise der Auswerteeinheit 15 zugeführt und zur Erzeugung von qualitätskennzeichnenden Größen herangezogen. Die Messwerte MW' werden auf der Basis der dielektrischen Eigenschaften des Messraumes 21' erzeugt. Die Erfassung dieser dielektrischen Eigenschaften erfolgt analog zur Funktionsweise der erfindungsgemäßen Vorrichtung 20. Es ergeben sich daher auch dieselben Vorteile. Zu bemerken ist allerdings, dass die Anordnung einer erfindungsgemäßen Vorrichtung 20' gerade im Bereich der Auslaufführung 6 von Vorteil ist, da üblicherweise die Geschwindigkeit des hier zu vermessenden Fasergemenges $FB_{ab}$ höher ist.

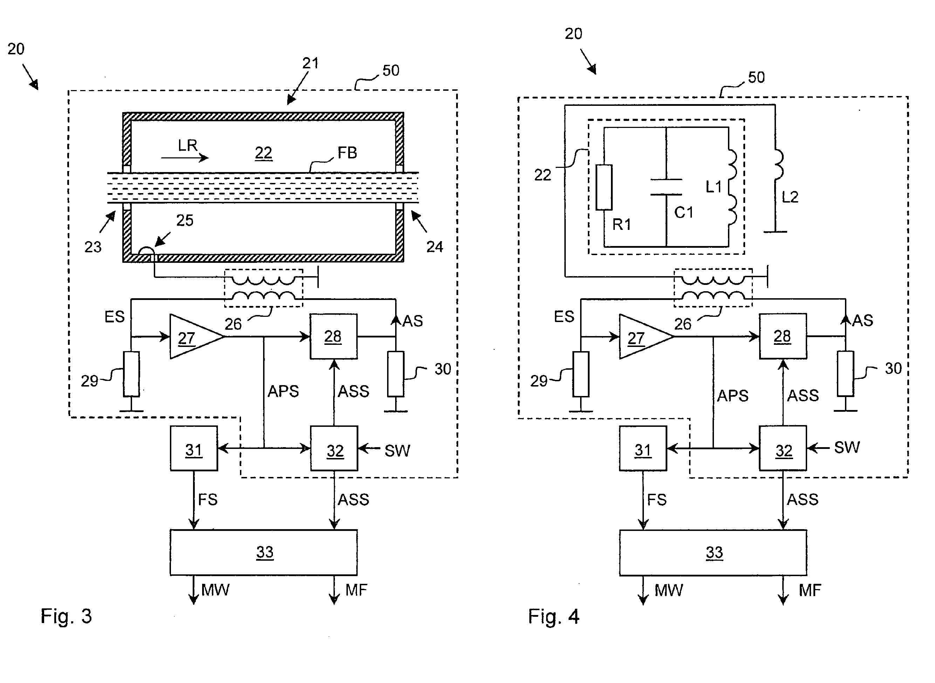

Figur 3 zeigt ein erstes Ausführungsbeispiel einer erfindungsgemäßen Vorrichtung 20. Sie umfasst einen Mikrowellenresonator 21 mit einem Messraum 22. Durch eine Eingangsöffnung 23 und durch eine Ausgangsöffnung 24 kann das zu vermessende Faserband FB in Laufrichtung LR durch den Messraum 22 kontinuierlich hindurchgefördert werden. Der Mikrowellenresonator 21 weist eine einzige Koppelanordnung 25 auf. Er ist somit als Eintorresonator ausgebildet. Bei der Koppelanordnung 25 kann es sich, wie in Figur 3 schematisch dargestellt, um eine induktive Koppelschleife handeln. Es sind jedoch auch andere Koppelanordnungen, wie z.B. kapazitive Koppelstifte möglich, ohne dass das etwas an der grundsätzlichen Funktion der erfindungsgemäßen Anordnung ändert. Die Koppelanordnung 25 verkoppelt den Resonator 21 und somit den Messraum 22 über einen Übertrager 26 mit dem Eingangssignal ES und mit dem Ausgangssignal AS einer aktiven Verstärkeranordnung 27, 28.

Die Kopplung über den Übertrager 26 ist beispielhaft anzusehen. Der Übertrager 26 kann zum Beispiel weggelassen werden, ohne dass das an der grundsätzlichen Funktion der Anordnung etwas ändert, wenn der Massepunkt in der Schaltung entsprechend verlagert wird.

Die aktive Verstärkeranordnung 27, 28 ist eingangsseitig mit einer Reaktanz 29 und ausgangsseitig mit einer Reaktanz 30 abgeschlossen. Sie umfasst einen Verstärker 27 mit einer konstanten Verstärkung und damit in Reihe geschaltet ein Amplitudenstellglied 28, dessen Verstärkung mittels eines Amplitudenstellesignals ASS einstellbar ist. Zwischen dem Verstärker 27 und dem Amplitudenstellglied 28 ist ein Amplitudensignal APS abgegriffen, welches wegen den konstanten Verstärkung proportional zum Eingangssignal ES ist. Das Amplitudensignal APS wird einer Frequenzmessanordnung 31 und einem Amplitudendetektor 32 zugeführt.

Die Frequenzmessanordnung 31 erzeugt aus dem Amplitudensignal APS ein Frequenzsignal FS, welches mit der Frequenz des Mikrowellenoszillators korrespondiert. Der Amplitudendetektor 32 hingegen vergleicht das Amplitudensignal APS mit einem vorgegebenen Sollwert SW und erzeugt hieraus das Amplitudenstellsignal ASS zur Steuerung der Verstärkung des Amplitudenstellgliedes 28. Das Amplitudenstellsignal ASS wird weiterhin an eine Signalauswertestufe 33 übertragen. Auch das Frequenzsignal FS wird von der Frequenzmessanordnung 31 an die Signalauswertestufe 33 übermittelt.

Die Signalauswertestufe 33 wertet die genannten Signal aus und erzeugt Messwerte MW, welche mit der längenspezifischen Masse des im Messraum 22 befindlichen Fasergemenges FD korrespondieren und Messwerte MF, welche mit der Feuchte des im Messraum 22 befindlichen Fasergemenges FB korrespondieren.

Figur 4 zeigt ein Ersatzschaltbild der anhand der Figur 3 erläuterten Vorrichtung 20. Die elektrische Wirkungsweise des Mikrowellenresonators 20 kann für eine bestimmte Resonanzfrequenz prinzipiell durch ein Ersatzschaltbild mit einem einzigen Parallelschwingkreis oder durch ein Ersatzschaltbild mit einem einzigen Serienschwingkreis dargestellt werden. Ob der Mikrowellenresonator 20 durch einen Serienschwingkreis oder durch einen Parallelschwingkreis beschrieben wird, ist letztlich eine Frage der Zweckmäßigkeit und hängt von der Art der Ankopplung ab.

Im folgenden werden ohne Einschränkung der Allgemeinheit Ersatzschaltungen mit Parallelschwingkreisen diskutiert. In bekannter Weise können dazu nach dem Dualitätsprinzip duale Schaltungen mit Serienschwingkreisen gefunden werden und auch induktive Ein- und/oder Auskoppelanordnungen durch kapazitive Ein- und/oder Auskopplungen ersetzt werden. Diese Variationen folgen einer wohlbekannten Systematik der Generierung von abgeleiteten Schaltungen.

Die elektrischen Eigenschaften des Mikrowellenresonators 21 können somit durch den Widerstand R1, die Kapazität C1 und die Induktivität L1, welche parallel geschaltet sind, dargestellt werden. Der Mikrowellenresonator 21 wird also in elektrischer Hinsicht durch den Parallelschwingkreis R1, C1, L1 dargestellt. Sowohl die Masse des Trockenanteils als auch die Masse des Feuchtanteils des im Messraum 22 befindlichen Fasergemenges FB beeinflussen den Wert des Widerstandes R1 und den Wert der Kapazität C1. Hierdurch wird sowohl die Güte als auch die Resonanzfrequenz des Parallelschwingkreises R1, C1, L1 verändert. Da jedoch der Einfluss des Trockenanteils des Faserbandes auf die Kenngrößen des Parallelschwingkreises R1, C1, L1 anderen Gesetzmäßigkeiten folgt als der Einfluss des Feuchtanteils des Faserbandes FB, kann aus der Kenntnis der Güte und der Resonanzfrequenz des Parallelschwingkreises R1, C1, L1 sowohl auf die längenspezifische Masse des Feuchtanteils als auch auf die längenspezifische Masse des Trockenanteils des im Messraum 22 befindlichen Fasergemenges FB geschlossen werden. Die in der Figur 3 gezeigte und dem Mikrowellenresonator 21 zugeordnete Koppelanordnung 25 ist in der Figur 4 durch die Induktivität L2 repräsentiert.

Kern der erfindungsgemäßen Vorrichtung 20 ist ein Mikrowellenoszillator 50. Er umfasst eine aktive Verstärkeranordnung 27, 28, welche über ein Rückkopplungsnetzwerk rückgekoppelt ist. Das Rückkopplungsnetzwerk umfasst im wesentlichen den Übertrager 26, die Induktivität L2 sowie den Parallelschwingkreis R1, C1, L1, der den Mikrowellenresonator 21 repräsentiert. Die Verstärkung des Rückkopplungsnetzwerkes ist bei der Resonanzfrequenz des Parallelschwingkreises R1, C1, L1 am größten, wobei der Mikrowellenoszillator 50 mit der Resonanzfrequenz des Parallelschwingkreises R1, C1, L1 schwingt. Der Parallelschwingkreis R1, C1, L1 wird daher auch als frequenzbestimmender Parallelschwingkreis bezeichnet.

Weiterhin ist die Verstärkung des Rückkopplungsnetzwerkes bei der Resonanzfrequenz des Parallelschwingkreises R1, C1, L1 von dessen Güte abhängig. Im Ausführungsbeispiel der Figur 4 wird nun die Amplitude des Eingangssignals ES der Verstärkeranordnung 27, 28 konstant gehalten, indem die Verstärkung des Amplitudenstellgliedes 28 geregelt wird. Dies wird erreicht, indem ein Amplitudenstellsignal APS, welches proportional dem Eingangssignal ES ist, durch den Amplitudendetektor 32 mit einem Sollwert SW verglichen wird. Ergibt sich eine Differenz, so wird die Verstärkung des Amplitudenstellgliedes 28 mittels des Amplitudenstellsignals ASS solange verändert, bis die Differenz verschwunden ist. Eine Verringerung der Güte des Parallelschwingkreises R1, C1, L1 wird dabei durch eine Erhöhung der Verstärkung des Amplitudenstellgliedes 28 ausgeglichen. Umgekehrt wird eine Erhöhung der Güte des Parallelschwingkreises R1, C1, L1 zu einer Verringerung der Verstärkung des Amplitudenstellgliedes 28, so dass die Gesamtverstärkung der aktiven Verstärkeranordnung 27, 28 und des Rückkopplungsnetzwerkes stets konstant ist. Dabei ist der Wert des Amplitudenstellsignals ASS ein Maß für die Güte des Parallelschwingkreises R1, C1, L1.

Im Ergebnis kann mit der dargestellten Vorrichtung 20 ein mit der Resonanzfrequenz des Mikrowellenresonators 21 korrespondierendes Signal FS und ein mit der Güte des Mikrowellenresonators 21 korrespondierendes Signal ASS erzeugt werden, ohne dass es wie bei bekannten Mikrowellenvorrichtungen der Aufnahme einer Resonanzkurve bedarf. Der in der Figur 4 dargestellte Mikrowellenoszillator 50 arbeitet dabei nach dem Prinzip der induktiven Dreipunktschaltung.

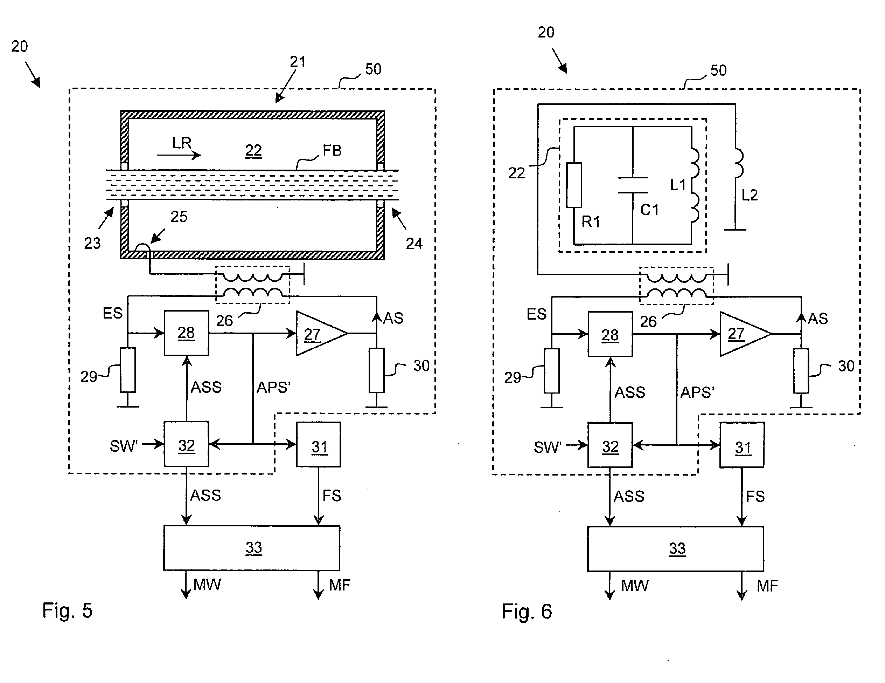

Figur 5 zeigt ein weiteres Beispiel einer erfindungsgemäßen Vorrichtung 20. Das zugehörige Ersatzschaltbild ist in Figur 6 dargestellt. Im Vergleich zu dem Ausführungsbeispiel der Figuren 3 und 4 ist die Reihenfolge des Verstärkers 27 und des Amplitudenstellgliedes 28 vertauscht. Das Amplitudensignal APS' ist proportional dem Ausgangssignal AS der Verstärkeranordnung 27, 28. Aus dem Amplitudensignal APS' und einem Sollwert SW' wird ein Amplitudenstellsignal ASS gebildet, welches zur Veränderung der Verstärkung des Amplitudenstellgliedes 28 herangezogen wird. Durch die gezeigte Schaltung ist sichergestellt, dass das Ausgangssignal der aktiven Verstärkeranordnung 27, 28 mit konstanter Amplitude schwingt. Auch bei einer Vorrichtung gemäß den Figuren 5 und 6 wird die Gesamtverstärkung der aktiven Verstärkeranordnung 27, 28 und des Rückkopplungsnetzwerkes konstant gehalten. So wird eine Verringerung der Verstärkung des Rückkopplungsnetzwerkes, welche durch eine Verringerung der Güte des Parallelschwingkreises R1, C1, L1 bewirkt wird, durch eine Erhöhung der Verstärkung des Amplitudenstellgliedes 28 ausgeglichen. Das hierzu erforderliche Amplitudenstellsignal ASS ist damit ein Maß für die Güte des Mikrowellenresonators 21.

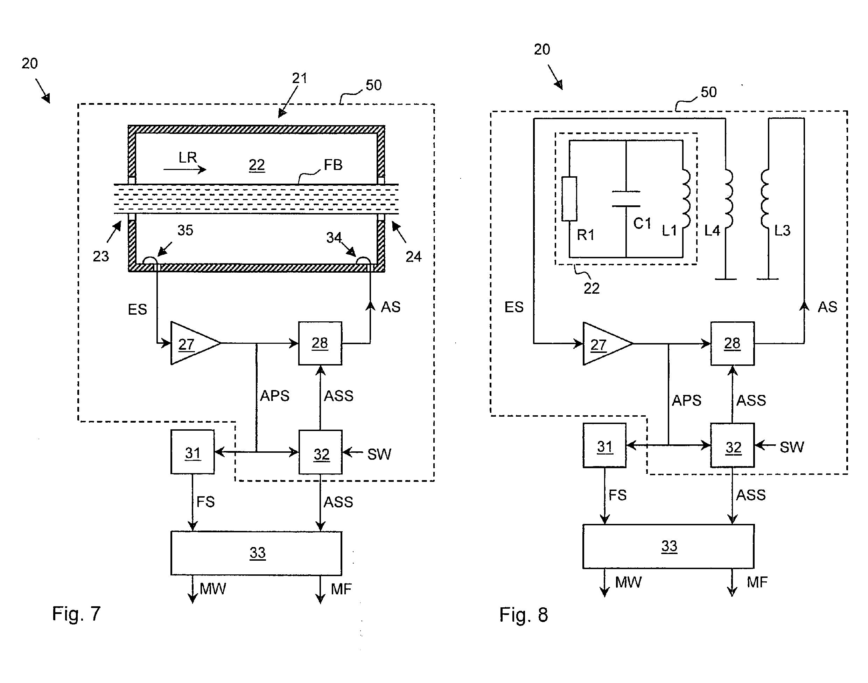

Figur 7 zeigt ein weiteres Ausführungsbeispiel einer erfindungsgemäßen Vorrichtung 20. Der Mikrowellenresonator 21 ist hier als Zweitorresonator ausgebildet. So ist dem Mikrowellenresonator 21 eine Einkoppelanordnung 34 zugeordnet, über die das Ausgangssignal AS der Verstärkeranordnung 27, 28 in den Messraum 22 eingekoppelt ist. Weiterhin ist dem Mikrowellenresonator 21 eine Auskoppelanordnung 35 zugeordnet, über die das Eingangssignal ES der Verstärkeranordnung 27, 28 aus dem Messraum 22 ausgekoppelt ist. Der weitere Aufbau der Anordnung entspricht im wesentlichen dem Ausführungsbeispiel der Figuren 3 und 4.

Im Ersatzschaltbild der Vorrichtung gemäß Figur 8 ist die Einkoppelanordnung 34 durch eine Induktivität L3 repräsentiert. Ebenso ist die Auskoppelanordnung 35 durch eine Induktivität L4 vertreten. Der hier dargestellte Mikrowellenoszillator 50 arbeitet nach dem Prinzip des Meissner-Oszillators. Das Rückkopplungsnetzwerk umfasst hier die Induktivität L3 und L4 und den Parallelschwingkreis R1, C1, L1. Auch bei einer derartigen Anordnung bestimmt die Resonanzfrequenz des Parallelschwingkreises R1, C1, L1 die Frequenz des Oszillators 50. Ebenso bestimmt die Güte des Parallelschwingkreises R1, C1, L1 die Verstärkung des Rückkopplungsnetzwerkes. In bereits anhand der Figuren 3 und 4 erläuterter Weise wird ein Frequenzsignal FS, welches mit der Resonanzfrequenz des Parallelschwingkreises R1, C1, L1 korrespondiert und ein Amplitudenstellsignal ASS, welches die Information über die Güte des Parallelschwingkreises R1, C1, L1 enthält, erzeugt. Aus diesen beiden Signalen FS und ASS werden dann Messwerte der längenspezifischen Masse und Messwerte der Feuchtigkeit MF des im Messraum 22 befindlichen Fasergemenges FB erzeugt.

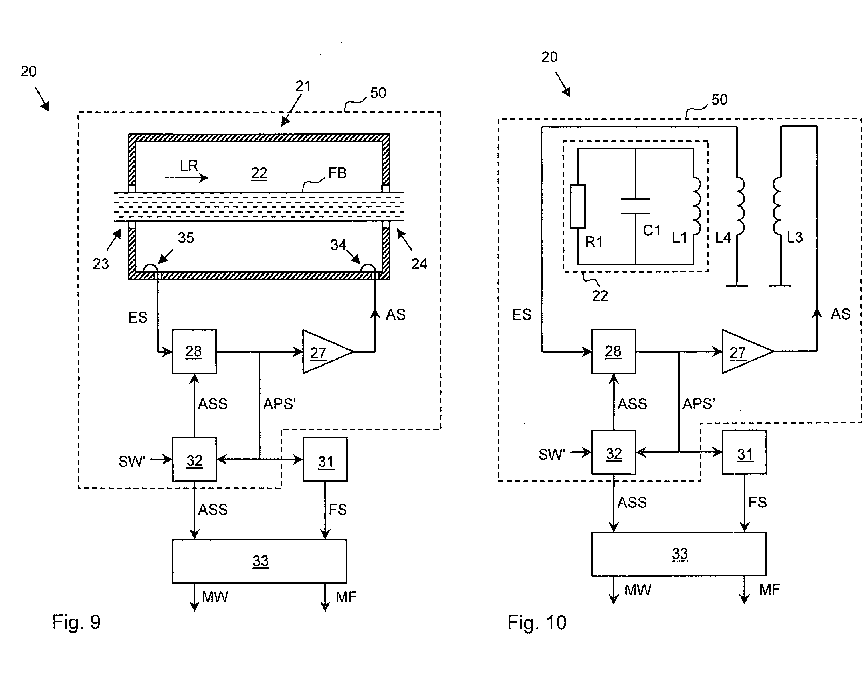

Die Figuren 9 und 10 zeigen ein weiteres Ausführungsbeispiel einer erfindungsgemäßen Vorrichtung 20, deren Oszillator 50 nach dem Prinzip des Meissner-Oszillators arbeitet. Wie im Ausführungsbeispiel der Figuren 5 und 6 ist hier jedoch das Amplitudenstellglied 28 eingangsseitig und der Verstärker 27 ausgangsseitig der aktiven Verstärkeranordnung 27, 28 angeordnet.

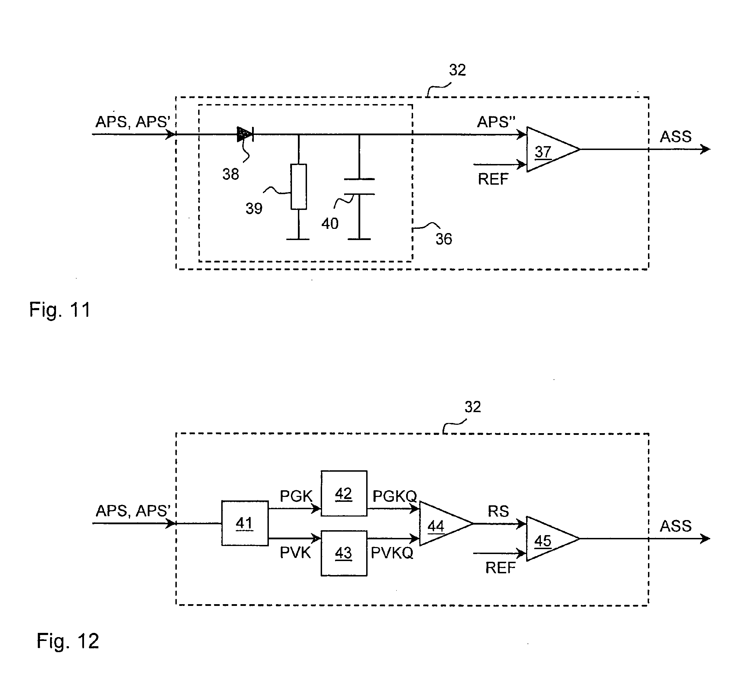

Figur 11 zeigt ein Ausführungsbeispiel eines Amplitudendetektors 32. Das Amplitudensignal APS bzw. das Amplitudensignal APS' wird zunächst mittels einer Gleichrichterschaltung 36 gleichgerichtet, so dass ein gleichgerichtetes Amplitudensignal APS" entsteht. Die Gleichrichterschaltung 36 ist als Einwegschaltung ausgebildet und umfasst eine Diode 38, einen Widerstand 39 und einen Kondensator 40. Die Gleichrichterschaltung 36 könnte jedoch auch als Mittelpunkt- oder als Brückenschaltung ausgeführt sein.

Das gleichgerichtete Amplitudensignal APS" wird mittels des Differenzverstärkers mit einem Referenzsignal REF verglichen, um so das Amplitudenstellsignal ASS zu erzeugen. Wird der gezeigte Amplitudendetektor 32 in einer der zuvor beschriebenen Vorrichtungen 20 zur Amplitudenregelung eingesetzt, so wird die Regelzeitkonstante durch die Zeitkonstante des aus dem Widerstand 39 und der Kapazität 40 bestehenden RC-Gliedes bestimmt. Durch eine entsprechende Dimensionierung des Widerstandes 39 und des Kondensators 40 kann die Zeitkonstante geringgehalten werden und so eine schnelle Regelung sichergestellt werden.

Figur 12 zeigt ein weiteres vorteilhaftes Ausführungsbeispiel eines Amplitudendetektors 32. Das Amplitudensignal APS bzw. APS' wird zunächst mittels eines Richtkopplers 41 in eine phasengleiche Komponente PGK (auch Kophasalkomponente genannt) und eine um 90° phasenverschobene Komponente PVK (auch Quadraturkomponente genannt) aufgeteilt. Die phasengleiche Komponente PGK wird mittels einer ersten Quadrierschaltung 42 quadriert, so dass ein quadriertes Signal PGKQ erzeugt ist. Die phasenverschobene Komponente PVK wird weiterhin mittels einer zweiten Quadrierschaltung 43 quadriert, so dass ein quadriertes Signal PVKQ entsteht. Das erste quadrierte Signal PGKQ und das zweite quadrierte Signal PVKQ werden dann mittels des Additionsverstärkers 44 addiert. Das hieraus resultierende Signal RS wird dann mittels des Differenzverstärkers 45 mit einem einstellbaren Referenzsignal REF verglichen. Am Ausgang des Differenzverstärkers 45 liegt dann das Amplitudenstellsignal ASS an.

Die Funktion dieser Schaltung lässt sich wie folgt erklären: Das Kophasalsignal PGK ist das Produkt einer zeitvariablen Amplitude, welche mit der Amplitude des Amplitudensignals APS, APS' korrespondiert, und einer Kosinusfunktion von $2\pi ft$, wobei f die Schwingfrequenz des Amplitudensignals APS, APS' und t die Zeit ist. Das Quadratursignal PVK hingegen ist das Produkt der zeitvariablen Amplitude und einer Sinusfunktion von $2\pi ft$. Quadriert man beide Signale PGK, PVK und addiert sie, so ist die Summe der Quadrate, nämlich das Signal RS, gleich dem Quadrat der zeitvariablen Amplitude. Bei der Addition haben sich die Quadrate der Kosinus- und der Sinus-Funktionen zum Wert 1 addiert. Dadurch wird das Quadrat der Amplitude gewonnen und der Träger eliminiert ohne dass eine Tiefpassfilterung oder eine andere frequenzabhängige Signalverarbeitung (abgesehen von dem vorgeschalteten Richtkoppler) erforderlich ist. Die Gewinnung des Quadrates der Amplitude erfolgt dadurch mit minimaler Zeitverzögerung. Das ist im Hinblick auf die Realisierung einer schnellen Regelung und die Erzielung kurzer Messzeiten vorteilhaft.

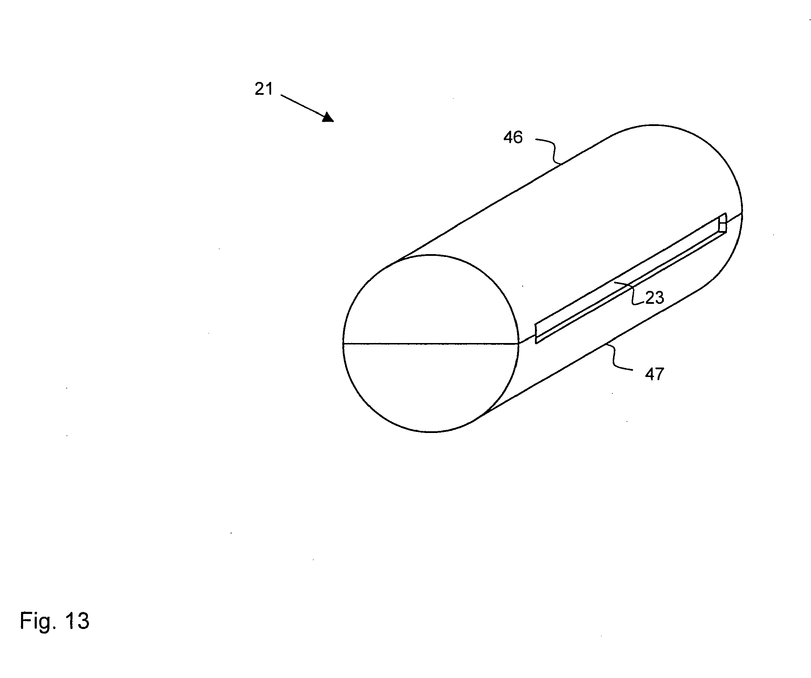

Figur 13 zeigt eine perspektivische Darstellung eines zweiteiligen Mikrowellenresonators 21. Dieser umfasst ein Resonatoroberteil 46 und ein Resonatorunterteil 47, welche im Betrieb des Mikrowellenresonators 21 durch geeignete Mittel verbunden sind. Die Eingangsöffnung 23 des Mikrowellenresonators 21 ist schlitzförmig ausgebildet. Sie ist so angeordnet, dass bei dem zur Messung verwendeten Schwingungsmodus des Mikrowellenresonators 21 die elektrischen Feldlinien des elektromagnetischen Feldes parallel zur Eingangsöffnung 23 sind, wobei die magnetischen Feldlinien dazu normal verlaufen. Hierdurch ist sichergestellt, dass eine Abstrahlung des elektromagnetischen Feldes über die Eingangsöffnung 23 minimiert ist. Die in der gezeigten Perspektive nicht sichtbare Ausgangsöffnung 24 liegt der Eingangsöffnung 23 gegenüber. Auch hier sind die elektromagnetischen Feldlinien parallel zur Ausgangsöffnung 24.

Die vorliegende Erfindung ist nicht auf die dargestellten und beschriebenen Ausführungsbeispiele beschränkt. Es sind Abwandlungen im Rahmen der Patentansprüche jederzeit möglich.

Die erfindungsgemäße Vorrichtung kann insbesondere zur Ermittlung der Faserbanddichte bzw. der längenspezifischen Masse des Faserbandes unter Berücksichtigung der Feuchte Verwendung finden. Die Vorrichtung kann zur Durchführung eines Algorithmus zur Trennung von Feuchte und Dichte anhand der Güte und der Frequenz des Mikrowellenresonators ausgebildet sein.

Der Mikrowellenresonator kann zumindest bereichsweise Materialien umfassen, die für Mikrowellen durchlässig sind (Keramik, Quarzglas) und deren Dielektrizitätekonstanten möglichst temperaturunabhängig sind. Dies betrifft insbesondere im Innern des Mikrowellenresonators angeordnete Elemente zum Führen des zu vermessenden Faserbandes.

Weiterhin kann der Mikrowellenresonator aus zwei im wesentlichen parallel angeordneten Halbzylindern ausgebildet sein. Dabei kann er, insbesondere wenn er für den Auslauf einer Karde oder Stecke vorgesehen ist, als Zylinder mit konzentrischer Durchführung für das Faserband ausgebildet sein. Er kann ferner, insbesondere wenn er für den Einlauf einer Stecke vorgesehen ist, einen Einführschlitz zum seitlichen Einführen des Faserbandes aufweisen.

Um eine Verfälschung der durch die Vorrichtung erzeugten Messwerte durch Temperatureinflüsse zu vermeiden, können folgende Maßnahmen vorgesehen sein: Ausbilden des Resonators aus Materialien mit geringem Wärmeausdehungskoefffizient (z.B Invar), Vorsehen von Mitteln zur thermischen Isolation des Resonators bzw. des gesamten Sensors einschließlich zumindest eines Teiles der an den Resonator angeschlossenen Beschaltung und/oder Vorsehen von Mitteln zur Beheizung (z. B. durch Heizfolien) oder Kühlung (z. B. durch Peltierelemente) des Resonators bzw. des Sensors.

Weiterhin können folgende Maßnahmen zur Kalibrierung der erfindungsgemäßen Vorrichtung vorgesehen sein: Durchführung der Kalibration des Sensors für verschiedene Faserbandmaterialien oder Faserbandmischungen anhand von Kalibrationskurven, die in Labormessungen in einer vorbetrieblichen Phase durch Ermittlung der Istbandfeinheit und/oder Ermittlung der Istfeuchte ermittelt wurden, Leeresonanzbestimmung, wobei Mittel verwendet werden, mit dem der Resonator vollständig von Faserband und eventuell angelagerten Schmutzresten gereinigt wird (Herausfördern des Faserbandes nach vorherigem Trennen, Ausblasen der Reste und Verschmutzungen).

Die erfindungsgemäße Textilmaschine kann insbesondere eine Karde oder Strecke sein, wobei eine erfindungsgemäße Vorrichtung als Auslaufsensor vorgesehen ist. Dabei kann die Vorrichtung am Streckwerksauslauf zwischen Auslaufzylinder und Kalanderwalzenpaar oder unmittelbar nach dem Kalanderwalzenpaar angeordnet sein. Mit Vorteil ist die Vorrichtung mit einem Qualitätsüberwachungssystem verbunden, wobei eine Absinken der Qualität des auslaufenden Faserbandes unter einen Schwellwert zu einer Abschaltung der Textilmaschine führt.

Insbesondere, wenn die erfindungsgemäße Textilmaschine eine Strecke ist, so kann eine erfindungsgemäße Vorrichtung als Einlaufsensor vorgesehen sein. Diese Vorrichtung kann unmittelbar vor dem Streckwerk oder in einem Einlaufbereich angeordnet und mit einer Reguliereinrichtung zum Ausregulieren von Schwankungen der längenspezifischen Masse des zu verstreckenden Faserbandes verbunden sein.

**Patentansprüche**

1. Vorrichtung (20, 20') für eine Textilmaschine (1), insbesondere für eine Spinnereivorbereitungsmaschine (1), beispielsweise für eine Strecke (1), Karde oder Kämmmaschine, oder für eine Spinnereimaschine, beispielsweise für eine Spinnmaschine oder Spulmaschine, zur Messung der längenspezifischen Masse und/oder der Feuchtigkeit eines strangförmigen, laufenden Fasergemenges (FB), mit einem Mikrowellenresonator (21, 21'), welcher einen Messraum (22) aufweist, durch den das Fasergemenge (FB) hindurchführbar ist, wobei ein Mikrowellenoszillator (50) vorgesehen ist, der eine aktive Verstärkeranordnung (27, 28) und ein der Verstärkeranordnung (27, 28) zugeordnetes Rückkopplungsnetzwerk mit einer frequenzbestimmenden Resonanzanordnung, welche einem LC-Schwingkreis (R1, C1, L1) äquivalent ist, aufweist, wobei der Mikrowellenresonator (21, 21') die frequenzbestimmende Resonanzanordnung des Mikrowellenoszillators (50) ist, sodass die Frequenz des Mikrowellenoszillators (50) die Resonanzfrequenz des Mikrowellenresonators (21, 21') ist, wobei eine Frequenzmessanordnung (31) zur Erzeugung eines mit der Frequenz des Mikrowellenoszillators (50) korrespondierenden Frequenzsignals (FS) vorgesehen ist, **gekennzeichnet durch** eine Anordnung zur Erfassung der Resonanzfrequenz und der Güte des Mikrowellenresonators (21, 21'), wobei die aktive Verstärkeranordnung (27, 28) ein Amplitudenstellglied (28) mit einer **durch** ein Amplitudenstellsignal (ASS) einstellbaren Verstärkung aufweist, wobei ein Amplitudendetektor (32) zur Erzeugung des Amplitudenstellsignals (ASS) vorgesehen ist, sodass die Gesamtverstärkung der aktiven Verstärkeranordnung (27, 28) und des Rückkopplungsnetzwerkes konstant gehalten ist, wobei eine Signalauswertestufe (33) vorgesehen ist, welche aus dem Frequenzsignal (FS) und dem Amplitudenstellsignal (ASS) Messwerte (MW, MW') der längenspezifische Masse und/oder Messwerte (MF) der Feuchtigkeit des strangförmigen, laufenden Fasergemenges (FB) erzeugt.

2. Vorrichtung (20, 20') nach Anspruch 1, **dadurch gekennzeichnet, dass** der Amplitudendetektor (32) zum Vergleichen der Amplitude eines mit dem Eingangssignal (ES) der aktiven Verstärkeranordnung (27, 28) korrespondierenden Amplitudensignals (APS) mit einem Sollwert (SW) ausgebildet ist, sodass das Eingangssignal (ES) der aktiven Verstärkeranordnung (27, 28) mit konstanter Amplitude schwingt.

3. Vorrichtung (20, 20') nach Anspruch 1, **dadurch gekennzeichnet, dass** der Amplitudendetektor (32) zum Vergleichen der Amplitude eines mit dem Ausgangssignal (AS) der aktiven Verstärkeranordnung (27, 28) korrespondierenden Amplitudensignals (APS') mit einem Sollwert (SW') ausgebildet ist, sodass das Ausgangssignal (AS) der aktiven Verstärkeranordnung (27, 28) mit konstanter Amplitude schwingt.

4. Vorrichtung (20, 20') nach einem der vorstehenden Ansprüche, **dadurch gekennzeichnet, dass** die aktive Verstärkeranordnung (27, 28) einen mit dem Amplitudenstellglied (28) in Reihe geschalteten Verstärker (27) mit konstanter Verstärkung aufweist.

5. Vorrichtung (20, 20') nach einem der vorstehenden Ansprüche, **dadurch gekennzeichnet, dass** der Mikrowellenoszillator (50) so ausgebildet ist, dass die aktive Verstärkeranordnung (27, 28) im amplitudenlinearen Bereich betrieben ist.

**6.** Vorrichtung (20, 20') nach einem der vorstehenden Ansprüche, **dadurch gekennzeichnet, dass** dem Mikrowellenresonator (21, 21') eine Koppelanordnung (25) zugeordnet ist, welche den Messraum (22) mit dem Eingangssignal (ES) und mit dem Ausgangssignal (AS) der aktiven Verstärkeranordnung (27, 28) verkoppelt.

**7.** Vorrichtung (20, 20') nach einem der vorstehenden Ansprüche, **dadurch gekennzeichnet, dass** der Mikrowellenoszillator (50) als Dreipunkt-Oszillator ausgebildet ist.

**8.** Vorrichtung (20, 20') nach einem der Ansprüche 1 bis 5, **dadurch gekennzeichnet, dass** dem Mikrowellenresonator (21, 21') eine Einkoppelanordnung (34), über die das Ausgangssignal (AS) der Verstärkeranordnung (27, 28) in den Messraum (22) eingekoppelt ist, und eine Auskoppelanordnung (35), über die das Eingangssignal (ES) der Verstärkeranordnung (27, 28) aus dem Messraum (22) ausgekoppelt ist, zugeordnet ist.

**9.** Vorrichtung (20, 20') nach vorstehendem Anspruch, **dadurch gekennzeichnet, dass** der Mikrowellenoszillator (50) als Meissner-Oszillator ausgebildet ist.

**10.** Vorrichtung (20, 20') nach einem der vorstehenden Ansprüche, **dadurch gekennzeichnet, dass** der Amplitudendetektor (32) eine Gleichrichterschaltung (36) zum Gleichrichten des Amplitudensignals (APS, APS') und einen Differenzverstärker (37) zur Erzeugung des Amplitudenstellsignals (ASS) anhand des gleichgerichteten Amplitudensignals (APS") und anhand eines vorgebbaren Referenzsignals (REF) aufweist.

**11.** Vorrichtung (20, 20') nach einem der Ansprüche 1 bis 9, **dadurch gekennzeichnet, dass** der Amplitudendetektor (32) eine Anordnung, vorzugsweise einen Richtkoppler (41), zum Auskoppeln einer phasengleichen Komponente (PGK) und einer um 90° phasenverschobenen Komponente (PVK) aus dem Amplitudensignal (APS), eine erste Quadrierschaltung (42) zum Quadrieren der phasengleichen Komponente (PGK), eine zweite Quadrierschaltung (43) zur Quadrierung der um 90° phasenverschobenen Komponente (PVK), einen Additionsverstärker (44) zum Erzeugen eines Proportionalsignals (PS), welches proportional der Summe aus der quadrierten phasengleichen Komponente (PGKQ) und der quadrierten phasenverschobenen Komponente (PVKQ) ist, und einen Differenzverstärker (45) zur Erzeugung des Amplitudenstellsignals (ASS) durch Vergleich des Proportionalsignals (PS) mit einem vorgebbaren Referenzsignal (REF) aufweist.

**12.** Vorrichtung (20, 20') nach einem der vorstehenden Ansprüche, **dadurch gekennzeichnet, dass** wenigstens ein FPGA vorgesehen ist.

**13.** Vorrichtung (20, 20') nach einem der vorstehenden Ansprüche, **dadurch gekennzeichnet, dass** der Mikrowellenresonator (21, 21') und zumindest Teile der daran angeschlossenen Schaltung (26, 27, 28, 29, 30, 31, 32, 33), insbesondere der Übertrager (26), der Verstärker (27), das Amplitudenstellglied (28), der eingangsseitige Abschlusswiderstand (29), der ausgangsseitige Abschlusswiderstand (30), die Frequenzmessanordnung (31), der Amplitudendetektor (32) und/oder die Signalauswertestufe (33), in einem gemeinsamen Gehäuse angeordnet sind.

**14.** Vorrichtung (20, 20') nach einem der vorstehenden Ansprüche, **dadurch gekennzeichnet, dass** eine Kommunikationsschnittstelle zum Anbinden der Vorrichtung an eine Kommunikationsverbindung, beispielsweise an einen CAN-Bus, einer Textilmaschine (1) vorgesehen ist.

**15.** Textilmaschine (1), insbesondere eine Spinnereivorbereitungsmaschine (1), beispielsweise eine Strecke (1), Karde oder Kämmmaschine, oder eine Spinnereimaschine, beispielsweise eine Spinnmaschine oder Spulmaschine, **dadurch gekennzeichnet, dass** wenigstens eine Vorrichtung (20, 20') nach einem der vorstehenden Ansprüche vorgesehen ist.

**Claims**

**1.** Device (20, 20') for a textile machine (1), in particular for a spinning preparation machine (1), such as a draw frame (1), carder or combing machine, or for a machine in a spinning mill, such as for a spinning machine or a winder, for measuring the mass per unit length and/or moisture of a skein-like, moving fibre batch (FB), with a microwave resonator (21, 21') that incorporates a measuring chamber (22) through which the fibre batch (FB) can be passed, wherein a microwave oscillator (50) is provided that incorporates an active amplifier arrangement (27, 28) and, associated with the amplifier arrangement (27, 28), a feedback network that includes a frequency-determining resonant arrangement that is equivalent to an LC resonant circuit (R1, C1, L1), wherein the microwave resonator (21, 21') is the frequency-determining resonant arrangement of the microwave oscillator (50), so that the frequency of the microwave oscillator (50) is the resonant frequency of the microwave resonator (21, 21'), wherein a frequency measuring arrangement (31) is provided to generate a frequency signal (FS) that corresponds to the frequency of the microwave oscillator (50), **characterized by** an arrangement for determining the resonant frequency and the quality factor of the microwave resonator (21, 21'), wherein the active amplifier arrangement (27, 28) incorporates an amplitude control element (28) having an amplification that can be adjusted by an amplitude control signal (ASS), whereby an amplitude detector (32) is provided to generate the amplitude control signal (ASS), so that the total amplification of the active amplifier arrangement (27, 28) together with the feedback network is held constant, wherein a signal evaluation stage (33) is provided which obtains measurements (MW, MW') of the mass per unit length and/or measurements (MF) of the moisture in the skein-like, moving fibre batch (FB) from the frequency signal (FS) and/or from the amplitude control signal (ASS).

2. Device (20, 20') according to Claim 1, **characterized in that** the amplitude detector (32) is designed to compare the amplitude of an amplitude signal (APS) that corresponds to the input signal (ES) of the active amplifier arrangement (27, 28) with a setpoint value (SW), so that the input signal (ES) of the active amplifier arrangement (27, 28) oscillates with constant amplitude.

3. Device (20, 20') according to Claim 1, **characterized in that** the amplitude detector (32) is designed to compare the amplitude of an amplitude signal (APS') that corresponds to the output signal (AS) of the active amplifier arrangement (27, 28) with a setpoint value (SW'), so that the output signal (AS) of the active amplifier arrangement (27, 28) oscillates with constant amplitude.

4. Device (20, 20') according to one of the foregoing claims, **characterized in that** the active amplifier arrangement (27, 28) incorporates an amplifier (27) with a constant amplification factor in series with the amplitude control element (28).

5. Device (20, 20') according to one of the foregoing claims, **characterized in that** the microwave oscillator (50) is designed in such a way that the active amplifier arrangement (27, 28) is operated within its linear amplitude range.

6. Device (20, 20') according to one of the foregoing claims, **characterized in that** a coupling arrangement (25) which couples the measuring chamber (22) to the input signal (ES) and to the output signal (AS) of the active amplifier arrangement (27, 28) is assigned to the microwave resonator (21, 21').

7. Device (20, 20') according to one of the foregoing claims, **characterized in that** the microwave oscillator (50) is constructed as a three-point oscillator.

8. Device (20, 20') according to one of Claims 1 to 5, **characterized in that** an inward coupling arrangement (34) is assigned to the microwave resonator (21, 21') by means of which inward coupling arrangement (34) the output signal (AS) of the amplifier arrangement (27, 28) is coupled into the measuring chamber (22), and an outward coupling arrangement (35) is assigned to the microwave resonator (21, 21') by means of which outward coupling arrangement (35) the input signal (ES) of the amplifier arrangement (27, 28) is coupled out of the measuring chamber (22).

9. Device (20, 20') according to the foregoing claim, **characterized in that** the microwave oscillator (50) is implemented as a Meissner oscillator.

10. Device (20, 20') according to one of the foregoing claims, **characterized in that** the amplitude detector (32) incorporates a rectifier circuit (36) to rectify the amplitude signal (APS, APS') and a differential amplifier (37) to generate the amplitude control signal (ASS) on the basis of the rectified amplified signal (APS") and of a specifiable reference signal (REF).

11. Device (20, 20') according to one of Claims 1 to 9, **characterized in that** the amplitude detector (32) incorporates an arrangement, favourably a hybrid coupler (41), for coupling out a component with an unchanged phase (PGK) and a component with a phase shifted by 90° (PVK) from the amplitude signal (APS), a first squaring circuit (42) for squaring the component of unchanged phase (PGK), a second squaring circuit (43) for squaring the component whose phase has been shifted by 90° (PVK), a summing amplifier (44) to generate a proportional signal (PS) that is proportional to the sum of the squared component of unchanged phase (PGKQ) as well as the squared phase-shifted component (PVKQ), and a differential amplifier (45) to generate the amplitude control signal (ASS) by comparing the proportional signal (PS) to a specifiable reference signal (REF).

12. Device (20, 20') according to one of the foregoing claims, **characterized in that** at least one FPGA is provided.

13. Device (20, 20') according to one of the foregoing claims, **characterized in that** the microwave resonator (21, 21') and at least part of the circuitry connected to it (26, 27, 28, 29, 30, 31, 32, 33), in particular the transformer (26), the amplifier (27), the amplitude control element (28), the termination resistor at the input (29), the termination resistor at the output (30), the frequency measuring arrangement (31), the amplitude detector (32) and/or the signal evaluation stage (33) are located in a common housing.

14. Device (20, 20') according to one of the foregoing claims, **characterized in that** a communication interface is provided for connecting the device to an external communication path, such as a CAN bus, on a textile machine (1).

15. Textile machine (1), in particular a spinning preparation machine (1), for instance a draw frame (1), carder or combing machine, or a machine in a spinning mill such as a spinning machine or a winder, **characterized in that** at least one device (20, 20') according to one of the foregoing claims is provided.


**Revendications**

1. Dispositif (20, 20') pour une machine textile (1), particulièrement une machine de préparation de filage (1), par exemple une étireuse (1), une cardeuse ou une peigneuse, ou pour une machine de filage, par exemple une machine à filer ou une machine à bobiner, pour mesurer la masse spécifique à la longueur et/ou l'humidité d'un mélange de fibres (FB) en translation en boyau continu, avec un résonateur à hyperfréquences (21, 21'), qui comporte un espace de mesure (22), à travers lequel le mélange de fibres (FB) peut être transporté, sachant qu'il est prévu un oscillateur à hyperfréquences (50), qui comporte un montage amplificateur (27, 28) actif et un réseau de rétrocouplage attribué au montage amplificateur (27, 28), avec un montage de résonance déterminant la fréquence, qui est équivalent à un circuit résonant LC (R1, C1, L1), sachant que le résonateur à hyperfréquences (21, 21') est le montage de résonance déterminant la fréquence de l'oscillateur à hyperfréquences (50), si bien que la fréquence de l'oscillateur à hyperfréquences (50) est la fréquence de résonance du résonateur à hyperfréquences (21, 21'), sachant qu'il est prévu un montage de mesure de fréquence (31) pour générer un signal de fréquence (FS) correspondant à la fréquence de l'oscillateur à hyperfréquences (50), **caractérisé par** un montage pour la saisie de la fréquence de résonance et de la qualité du résonateur à hyperfréquences (21, 21'), sachant que le montage amplificateur actif (27, 28) présente un élément de commande d'amplitude (28) avec une amplification réglable au moyen d'un signal de commande d'amplitude (ASS), sachant qu'il est prévu un détecteur d'amplitude (32) pour générer le signal de commande d'amplitude (ASS), si bien que l'amplification globale du montage amplificateur actif (27, 28) et du réseau de rétrocouplage est maintenue à une valeur constante, sachant qu'il est prévu une étage d'évaluation de signaux (33), qui génère à partir du signal de fréquence (FS) et du signal de commande d'amplitude (ASS) des valeurs de mesure (MW, MW') de la masse spécifique à la longueur et/ou des valeurs de mesure (MF) de l'humidité du mélange de fibres en translation en boyau continu (FB).

2. Dispositif (20, 20') selon la revendication 1, **caractérisé en ce que** le détecteur d'amplitude (32) se présente sous une forme lui permettant de comparer l'amplitude d'un signal d'amplitude (APS) correspondant au signal d'entrée (ES) du montage amplificateur actif (27, 28) avec une valeur de consigne (SW), si bien que le signal d'entrée (ES) du montage amplificateur actif (27, 28) oscille avec une amplitude constante.

3. Dispositif (20, 20') selon la revendication 1, **caractérisé en ce que** le détecteur d'amplitude (32) se présente sous une forme lui permettant de comparer l'amplitude d'un signal d'amplitude (APS') correspondant au signal de sortie (AS) du montage amplificateur actif (27, 28) avec une valeur de consigne (SW'), si bien que le signal de sortie (AS) du montage amplificateur actif (27, 28) oscille avec une amplitude constante.

4. Dispositif (20, 20') selon l'une quelconque des revendications précédentes, **caractérisé en ce que** le montage amplificateur actif (27, 28) présente un amplificateur (27) avec amplification constante, couplé en série avec l'élément de commande d'amplitude (28).

5. Dispositif (20, 20') selon l'une quelconque des revendications précédentes, **caractérisé en ce que** l'oscillateur à hyperfréquences (50) se présente sous une forme telle que le montage amplificateur actif (27, 28) soit exploité dans une plage linéaire en amplitude.

6. Dispositif (20, 20') selon l'une quelconque des revendications précédentes, caractérisé en ce qu'un montage de couplage (25) est attribué au résonateur à hyperfréquences (21, 21'), qui couple l'espace de mesure (22) avec le signal d'entrée (ES) et avec le signal de sortie (AS) du montage amplificateur actif (27, 28).

7. Dispositif (20, 20') selon l'une quelconque des revendications précédentes, **caractérisé en ce que** l'oscillateur à hyperfréquences (50) se présente sous la forme d'un oscillateur à trois positions.

8. Dispositif (20, 20') selon l'une quelconque des revendications 1 à 5, **caractérisé en ce qu'au** résonateur à hyperfréquences (21, 21') sont attribués un montage de couplage (34), par lequel le signal de sortie (AS) du montage amplificateur (27, 28) est couplé dans l'espace de mesure (22), et un montage de découplage (35), par lequel le signal d'entrée (ES) du montage amplificateur (27, 28) est découplé de l'espace de mesure (22).

9. Dispositif (20, 20') selon la revendication précédente, **caractérisé en ce que** l'oscillateur à hyperfréquences (50) se présente sous la forme d'un oscillateur de Meissner.

10. Dispositif (20, 20') selon l'une quelconque des revendications précédentes, **caractérisé en ce que** le détecteur d'amplitude (32) comporte un montage redresseur (36) pour redresser le signal d'amplitude (APS, APS') et un amplificateur différentiel (37) pour générer le signal de commande d'amplitude (ASS) à partir du signal d'amplitude redressé (APS") et à partir d'un signal de référence (REF) allouable.

11. Dispositif (20, 20') selon l'une quelconque des revendications 1 à 9, **caractérisé en ce que** le détecteur d'amplitude (32) comporte un montage, de préférence un coupleur directif (41), pour découpler une composante en phase (PGK) et une composante déphasée de 90° (PVK) du signal d'amplitude (APS), un premier circuit de mise au carré (42) pour mettre au carré le composant en phase (PGK), un second circuit de mise au carré (43) pour mettre au carré le composant déphasé de 90°, un amplificateur d'addition (44) pour générer un signal proportionnel (PS), qui est proportionnel à la somme de la composante en phase mise au carré (PGKQ) et de la composante déphasée mise au carré (PVKQ), et un amplificateur différentiel (45) pour générer le signal de commande d'amplitude (ASS) par comparaison du signal proportionnel (PS) avec un signal de référence allouable (REF).

12. Dispositif (20, 20') selon l'une quelconque des revendications précédentes, **caractérisé en ce qu'**il est prévu au moins un FPGA.

13. Dispositif (20, 20') selon l'une quelconque des revendications précédentes, **caractérisé en ce que** le résonateur à hyperfréquences (21, 21') et au moins des parties du circuit (26, 27, 28, 29, 30, 31, 32, 33), qui y est raccordé, en particulier le transducteur (26), l'amplificateur (27), l'élément de commande d'amplitude (28), la résistance terminale côté entrée (29), la résistance terminale côté sortie (30), le montage de mesure de fréquence (31), le détecteur d'amplitude (32) et/ou l'étage d'évaluation de signaux (33) sont logés dans un boîtier commun.

14. Dispositif (20, 20') selon l'une quelconque des revendications précédentes, **caractérisé en ce qu'**il est prévu une interface de communication pour intégrer le dispositif dans une liaison de communication, par exemple un bus CAN, d'une machine textile (1).

15. Machine textile (1), en particulier une machine de préparation de filage (1), par exemple une étireuse (1), une cardeuse ou une peigneuse, ou une machine de filage, par exemple une machine à filer ou une machine à bobiner, **caractérisée en ce qu'**il est prévu au moins un dispositif (20, 20') selon l'une quelconque des revendications précédentes.

Fig. 1

Fig. 2

Fig. 3

Fig. 4

EP 2 069 561 B1

18

Fig. 6

Fig. 5

Fig. 8

Fig. 7

Fig. 9

Fig. 10

EP 2 069 561 B1

EP 2 069 561 B1

APS, APS'  →  38  39  40  36  APS''  →  37  REF  →  ASS →

32

Fig. 11

APS, APS'  →  41  PGK →  42  PGKQ →  44  RS →  45  ASS →
                    PVK →  43  PVKQ →       REF →

32

Fig. 12

Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 6476619 B

### In der Beschreibung aufgeführte Nicht-Patentliteratur

- **BUCH U. TIETZE ; CH. SCHENK.** Halbleiter-Schaltungstechnik. 1978, 419